# EUROPEAN PATENT APPLICATION

(11) **EP 2 716 760 A1**
(43) Date of publication of application: **09.04.2014**
(21) Application number: 12793453.7
(22) Date of filing: 01.06.2012
(51) Int. Cl.: C12N 15/09, C12N 1/21

(54) **PRIMER SET AND METHOD FOR HOMOLOGOUS RECOMBINATION**

(30) Priority: 02.06.2011 JP 2011124459
(71) Applicant: Mitsui Engineering and Shipbuilding Co, Ltd., Tokyo 104-8439 (JP); Hiroshima University, Higashi-Hiroshima-shi Hiroshima 739-8511 (JP); Gifu University, Gifu-shi, Gifu 501-1193 (JP)
(72) Inventor: NAKASHIMADA Yutaka, Higashihiroshima-shi Hiroshima 739-8530 (JP); KITA Akihisa, Higashihiroshima-shi Hiroshima 739-8530 (JP); SUZUKI Tohru, Gifu City Gifu 501-1193 (JP); SAKAI Shinsuke, Ichihara-shi Chiba 290-8531 (JP); TAKAOKA Kazue, Tokyo 104-8439 (JP)
(74) Representative: Laufhütte, Dieter
(86) International application number: PCT/JP2012/064307
(87) International publication number: WO 2012/165627

(57) **Abstract**

The present invention provides a primer set used for transformation that imparts a uracil requiring property by deleting or destroying a gene coding for orotidine-5-phosphate decarboxylase in Moorella bacteria.

The present invention is accomplished by a primer set that is used for creating a uracil requiring strain obtained by deleting or destroying a gene coding for orotidine-5-phosphate decarboxylase in Moorella bacteria by homologous recombination and is represented by SEQ ID No. 1 and 2 that amplify an upstream region adjacent to said gene coding for orotidine-5-phosphate decarboxylase.

SEQUENCE LISTING

<110> MITSUI ENGINEERING & SHIPBUILDING Co., LTD
<110> Hiroshima University
<110> Gifu University

<120> Primer sets and methods for homologous recombination

<130> P12021-000

<160> 18

<210> 1
<211> 33
<212> DNA
<213> Artificial Sequence

<220>
<223> primer

<400> 1
tgacgttcta gaccctacct ctccaagatt acc 33

<210> 2
<211> 29
<212> DNA
<213> Artificial Sequence

<220>
<223> primer

<400> 2
tgacgtacta gtggcaagca ggccagaag 29

<210> 3
<211> 33
<212> DNA
<213> Artificial Sequence

<220>
<223> primer

<400> 3
tgacgtacta gtaacttcgg cctgctttca tgc 33

<210> 4
<211> 34
<212> DNA
<213> Artificial Sequence

<220>
<223> primer

<400> 4
tgacctgata tctgtccaag cttatgcacc ttcc 34

<210> 5
<211> 22
<212> DNA
<213> Artificial Sequence

<220>
<223> primer

<400> 5
accctacctc tccaagatta cc 22

<210> 6
<211> 22
<212> DNA
<213> Artificial Sequence

<220>
<223> primer

<400> 6
tgtccaagct tatgcacctt cc 22

<210> 7
<211> 19
<212> DNA
<213> Artificial Sequence

<220>
<223> primer

<400> 7
tgtcctcaac accctcacc 19

<210> 8
<211> 19
<212> DNA
<213> Artificial Sequence

<220>
<223> primer

<400> 8
tcttcccagg tcctgtagg 19

<210> 9
<211> 21
<212> DNA
<213> Artificial Sequence

<220>
<223> primer

<400> 9
acctgaagtt ccacgacatc c 21

<210> 10
<211> 18
<212> DNA
<213> Artificial Sequence

<220>
<223> primer

<400> 10
ggtcacgatg acgaactc 18

<210> 11
<211> 35
<212> DNA
<213> Artificial Sequence

<220>
<223> primer

<400> 11
agactaacaa cttcaagggg tacatactcc tgcag 35

<210> 12
<211> 33
<212> DNA
<213> Artificial Sequence

<220>
<223> primer

<400> 12
cagtggttac tgacatcccg tccttaacgg agg 33

<210> 13
<211> 33
<212> DNA
<213> Artificial Sequence

<220>
<223> primer

<400> 13
agactaacaa cttcagctcc tccgttaagg acg 33

<210> 14
<211> 33
<212> DNA
<213> Artificial Sequence

<220>
<223> primer

<400> 14
cagtggttac tgacacttca agcaccaggc gag 33

<210> 15
<211> 33
<212> DNA
<213> Artificial Sequence

<220>
<223> primer

<400> 15
agactaacaa cttcactcgc ctggtgcttg aag 33

<210> 16
<211> 34
<212> DNA
<213> Artificial Sequence

<220>
<223> primer

<400> 16
cagtggttac tgacaggaag ggaagggctt ttcc 34

<210> 17

<211> 24
<212> DNA
<213> Artificial Sequence

<220>
<223> primer

<400> 17
tgaagttgtt agtcttcctc atgc 24

<210> 18
<211> 22
<212> DNA
<213> Artificial Sequence

<220>
<223> primer

<400> 18
gtcagtaacc actgatgaac ac 22

## Description

### Technical field

The present invention relates to a primer set used for a process for expressing a transforming-gene in Moorella bacteria by homologous recombination and a method for homologous recombination by using the primer set, particularly a primer set and a method for homologous recombination by using the primer set used for transformation by deleting or destroying a gene coding for orotidine-5-phosphate decarboxylase in Moorella bacteria and imparting a uracil requiring property.

### Background art

Moorella bacteria, which are industrially advantageous in producing acetic acid and ethanol from a gas, are expected to show improvement in production efficiency for e.g. ethanol.

Inventors of the present invention examined introduction of a useful function such as improvement in production efficiency for ethanol by transforming Moorella bacteria. However, since Moorella bacteria are genetically specific unlike other types of bacteria and have not been fully identified in property, it is, in fact, technologically difficult to transform Moorella bacteria. In cases where a mutation treatment is performed on Moorella bacteria with a chemical substance such as nitrosoguanidine (NTG), a strain that can maintain ethanol production in large volumes even after several passages cannot be obtained, or a transformation-confirmed strain was not obtained when a plasmid vector as an extrachromosomal gene was attempted to be introduced.

Inventors of the present invention have experimentally succeeded in obtaining uracil-requiring Moorella bacteria by destroying a gene coding for orotate phosphoribosyl transferase (pyrE) as an enzyme associated with a uracil biosynthetic system by homologous recombination by using a Moorella sp. HUC22-1 strain

(Moorella bacteria) as a basal strain (Patent Document 1).

However, since the uracil-requiring Moorella bacteria had a difficulty in completing complementary sequence by incorporating a pyrE again, a specific method for expressing a transforming-gene by introducing the transforming-gene was unsuccessfully established.

### Prior art document

### Patent Document

Patent Document 1: JP-A-2010-17131

### Summary of the invention

### Problems to be solved by the invention

Inventors of the present invention carried out extended research, in order to establish a process for expressing a transforming-gene in Moorella bacteria by homologous recombination, obtain new uracil-requiring Moorella bacteria by destroying a gene coding for orotidine-5-phosphate decarboxylase (pyrF) and find out a specific method for expressing a transforming-gene by introducing a pyrF and a transforming-gene to a chromosome of the uracil-requiring Moorella bacteria to complete the present invention.

The present invention provides a primer set used for transformation by deleting or destroying a gene coding for orotidine-5-phosphate decarboxylase in Moorella bacteria and imparting a uracil requiring property and a method for homologous recombination by using the primer set.

Another problem of the present invention is obviously described as follows.

### Means for solving the problems

The above problems are solved by each of the following inventions.
1. A primer set used for creating a uracil requiring strain obtained by deleting or destroying a gene coding for orotidine-5-phosphate decarboxylase in Moorella bacteria by homologous recombination, wherein the primer set is represented by SEQ ID No. 1 and 2 that amplify an upstream region adjacent to said gene coding for orotidine-5-phosphate decarboxylase.
2. A primer set used for creating a uracil requiring strain obtained by deleting or destroying a gene coding for orotidine-5-phosphate decarboxylase in Moorella bacteria by homologous recombination, wherein, the primer set is represented by SEQ ID No. 3 and 4 that amplify a downstream region adjacent to said gene coding for orotidine-5-phosphate decarboxylase.
3. A primer set used for creating a uracil requiring strain obtained by deleting or destroying a gene coding for orotidine-5-phosphate decarboxylase in Moorella bacteria by homologous recombination, wherein the primer set comprises: a primer that is represented by SEQ ID No. 1 and 2 that amplify an upstream region adjacent to said gene coding for orotidine-5-phosphate decarboxylase; and a primer that is represented by SEQ ID No. 3 and 4 that amplify a downstream region adjacent to said gene coding for orotidine-5-phosphate decarboxylase.
4. A method for homologous recombination, comprising creating a uracil requiring strain obtained by deleting or destroying a gene coding for orotidine-5-phosphate decarboxylase in Moorella bacteria by homologous recombination by using the primer set according to any of 1 to 3.

### Advantageous effect of the invention

The present invention can provide a primer set used for transformation by deleting or destroying a gene coding for orotidine-5-phosphate decarboxylase in Moorella bacteria and imparting a uracil requiring property and a method for homologous recombination by using the primer set.

### Brief description of the drawings

Fig. 1 shows a location of each primer,
Fig. 2 shows a method for constructing a pyrF gene-destroying-vector pk18-dpryF,
Fig. 3 shows a location of each primer and a length of a DNA fragment,
Fig. 4 shows the results of a pyrF complementary potential strain confirmed by direct PCR,
Fig. 5 shows a scheme of a plasmid to be constructed,
Fig. 6 shows a sequence of a pyrF gene upstream region and a location of a primer,
Fig. 7 shows the results confirmed by KpnI treatment,
Fig. 8 shows the results of a colony direct PCR,
Fig. 9 shows a location of a restriction enzyme KpnI site, and
Fig. 10 shows the results of a colony direct PCR product treated with a restriction enzyme KpnI.

### Best mode for carrying out the invention

The present invention will be described as follows.

The primer according to the present invention can be used for a process for expressing a transforming-gene in Moorella bacteria by homologous recombination.

The Moorella bacteria are not particularly restricted, but preferred illustrative example thereof includes Moorella thermlacetia, M. thermoautotrophica and M. glycerini, and Moorella bacteria capable of producing ethanol from hydrogen and carbon dioxide or carbon monoxide in order to create a strain having a high ethanol production efficiency, and a Moorella thermlacetica ATCC 39073 strain.

Uracil requiring property means a property of a strain to require uracil for growth as a source of nutrition. Since Moorella bacteria can normally biosynthesize uracil, it has no uracil requiring property. However, in case of mutation that fails to biosynthesize uracil, uracil is required to be produced. A uracil requiring strain is a strain having this type of uracil requiring property.

An orotidine-5-phosphate decarboxylase is an enzyme involved in biosynthesizing UMP (uridine phosphate) as a precursor of a pyrimidine base required for biosynthesizing uracil. A strain characterized by lack of a gene coding therefor is unable to biosynthesize UMP, serving as a strain having a uracil requiring property.

A primer set represented by SEQ ID No. 1 to 4 in the present invention is used for homologous recombination that imparts a uracil requiring property by deleting or destroying a gene coding for orotidine-5-phosphate decarboxylase (hereinafter referred to as pyrF) in Moorella bacteria as a basal strain.

Specifically, a primer set represented by SEQ ID No. 1 and 2 amplifies an upstream region adjacent to a pyrF on a chromosome of Moorella bacteria.

Meanwhile, a primer set represented by SEQ ID No. 3 and 4 amplifies a downstream region adjacent to a pyrF on a chromosome of Moorella bacteria.

By ligating a PCR product obtained by PCR by using a primer set represented by SEQ ID No. 1 and 2 and each PCR product obtained by PCR by using a primer set represented by SEQ ID No. 3 and 4, a DNA fragment in which a pyrF is deleted or destroyed and an upstream region and a downstream region adjacent to the pyrF are contained is obtained.

A DNA fragment obtained is incorporated into a plasmid to construct a plasmid for destroying a pyrF, and introduced to Moorella bacteria as a basal strain. Accordingly, homologous recombination is induced on the basal strain and a pyrF is deleted or destroyed in a gene on a chromosome.

In the present invention, a method for introducing a plasmid for destroying a pyrF to Moorella bacteria is not particularly restricted, and preferably by means of e.g. electroporation.

In the above manner, the uracil-requiring Moorella bacteria (pyrF-destroying strain) are obtained by deleting or destroying a pyrF by homologous recombination.

Destruction of the pyrF gene can be confirmed by PCR by using a genome DNA of the Moorella bacteria as a basal strain and a genome DNA of the pyrF-destroying strain, each as a template.

A Primer Represented by SEQ ID No. 5 to 10 can preferably be used when PCR confirms if a pyrF in a created cell is deleted or destroyed.

In a pyrF-destroying strain obtained, a possibility of eliminating a uracil requiring property by introducing a pyrF gene and restoring a lost pyrimidine biosynthetic pathway, i.e, completing complementary sequence can be confirmed.

In order to confirm a complementary sequence, a gene coding for orotidine-5-phosphate decarboxylase on a chromosome in Moorella bacteria and an upstream region and a downstream region adjacent to the same are first amplified by using a Primer Represented by SEQ ID No. 1 and/or 4.

Subsequently, a DNA fragment amplified is incorporated into a plasmid to construct a plasmid for confirming a complementary sequence having a pyrF between homologous sites composed of an upstream region and a downstream region.

If a plasmid for confirming a complementary sequence is introduced to a pyrF-destroying strain to confirm bacteria growth in a uracil-defective medium, it is confirmed that the pyrF-destroying strain can be in the form of complementary sequence.

A uracil-requiring strain obtained by destroying a pyrF gene of a Moorella thermoacetica ATCC 39073 strain as a basal strain by using a Primer Represented by SEQ ID No. 1 to 4 in the present invention is under national deposit of NITE Patent Microorganisms Depositary (NPMD) as an MTA-D-pF strain (accession number: NITE P-1057). The national deposit will be transferred to international deposit as of June 1, 2012 and NPMD issues the certification "Notice of acceptance of biological genetic resources" as of this date. The international deposit accession number will be NITE BP-1057.

The cultural properties of the uracil-requiring Moorella bacteria are as follows.

A circular colony 3 to 5mm in diameter is formed in a modified ATCC 1754 PETC agar medium (*1) on an anaerobic condition at 55°C for 3 to 5 days.

i) Color: brown
ii) Surface shape: smooth
iii) Transparency: opaque

*1 Modified ATCC 1754 PETC agar medium

| | |
|---|---|
| NH₄Cl | 1.0g |
| KCl | 0.1g |
| MgSO₄ | 0.2g |
| NaCl | 0.8g |
| KH₂PO₄ | 0.1g |
| CaCl₂ | 0.02g |
| Yeast Extract | 1.0g |
| Uracil (No Yeast Extract added) | 0.01g |
| NaHCO₃ | 2.0g |
| Cysteine-HCl | 0.3g |
| Trace element solution (I) | 10ml |
| Vitamin solution (II) | 10ml |
| Distilled water | 1000ml |
| Agar (at high temperature) | 20g |
| Fructose | 5.0g |
| | pH 5.9 (prior to sterilization) |

| | |
|---|---|
| Sterilization temperature and time: 121°C, 15 min. | |

The compositions of the above (I) Trace element solution and (II) Vitamin solution are as follows.

| (I) Trace element solution | |
|---|---|
| Nitrolotriacetic acid | 2.0g |
| MnSO₄·H₂O | 1.0g |
| Fe(SO₄)₂(NH₄)₂·6H₂O | 0.8g |
| CoCl₂·6H₂O | 0.2g |
| ZnSO₄·7H₂O | 0.0002g |
| CuCl₂·2H₂O | 0.02g |
| NiCl₂·6H₂O | 0.02g |
| Na₂MlO₄·2H₂O | 0.02g |
| Na₂SeO₄ | 0.02g |
| Na₂WO₄ | 0.02g |
| Distilled water | 1000ml |
| | |

| (II) Vitamin solution | |
|---|---|
| Biotin | 2.0mg |
| Folic acid | 2.0mg |
| Pyridoxine-HCl | 10mg |
| Thiamine-HCl | 5.0mg |
| Riboflavin | 5.0mg |
| Nicotinic acid | 5.0mg |
| D-Ca-pantothenate | 5.0mg |
| Vitamin B₁₂ | 0.1mg |
| p-Aminobenzoic acid | 5.0mg |
| Thioctic acid | 5.0mg |
| Distilled water | 1000ml |

The pyrF-destroying strain obtained can preferably be used as a transforming basal strain that introduces a gene imparting a specific function such as improvement in ethanol productivity with reference to identified homologous sites and destroying-gene.

For instance, a vector for introducing a transforming-gene having a pyrF gene between 2 homologous sites and a transforming-gene to be introduced (gene that imparts a specific function) is prepared. Introduction of the same to a pyrF-destroying strain restores a pyrimidine biosynthetic pathway and eliminates a uracil requiring property, thereby forming Moorella bacteria that can express a specific transforming-gene. By isolating bacteria according to a uracil requiring property such as isolating bacteria that can grow in a uracil-defective medium, a transforming-gene is introduced to readily obtain a transforming strain that is imparted with a specific function.

In order to create a vector for introducing a transforming-gene, a primer set represented by SEQ ID No. 1 and 4 can preferably be used.

More specifically, a primer set represented by SEQ ID No. 1 and 4 amplifies a gene coding for orotidine-5-phosphate decarboxylase on a chromosome in Moorella bacteria and both regions adjacent to an upstream region and a downstream region.

By using a primer set represented by SEQ ID No. 1 and 4, a transforming-gene is incorporated into an upstream region or a downstream region of a pyrF in a DNA fragment amplified with a chromosome in Moorella as a template to prepare a DNA fragment in which a pyrF and a transforming-gene are present between both regions adjacent to the upstream region and the downstream region.

By incorporating a DNA fragment prepared into a plasmid, a vector for introducing a transforming-gene can be constructed.

In the present invention, a transforming-gene may be a gene that is originally found in Moorella bacteria, in addition to a gene that is not originally found in Moorella bacteria. More specifically, it is preferable that a gene that is originally found in Moorella bacteria be additionally incorporated in order to increase the number of genes retained (i.e. to promote expression).

By introducing a vector for introducing a transforming-gene to a pyrF-destroying strain, homologous recombination is induced to incorporate a pyrF and a transforming-gene into a chromosome of a pyrF-destroying strain.

In the above manner, transforming-gene-introduced Moorella bacteria obtained by introducing a transforming-gene by homologous recombination are obtained on a chromosome of a pyrF-destroying strain.

The transforming-gene-introduced Moorella bacteria can retain a transforming-gene on a chromosome so as to be expressed.

### EXAMPLE

### (Example 1)

The following media used for preparing Moorella bacteria and reagents are all prepared on an anaerobic and sterile condition and procedures are performed in an anaerobic environment.

### 1. Preparation of pyrF gene-destroying strain (ΔpyrF strain) of Moorella thermoacetica ATCC 39073 strain

### 1.1. Construction of pyrF gene-destroying-vector

A vector for destroying an orotidine-5'-phosphate decarboxylase gene pyrF of a Moorella thermoacetica ATCC 39073 strain was constructed according to the following procedures.

### [Construction of pyrF gene-destroying-vector pk18-dpryF]

First, PCR is performed on the conditions shown in Table 2 to amplify about 1000 bp of upstream and downstream regions of a pyrF gene by using a primer combination: a pyrF-uP-F1 (SEQ ID No. 1) and a pyrF-uP-R1 (SEQ ID No. 2), and a primer combination: a pyrF-dn-F1 (SEQ ID No. 3) and a pyrF-dn-R1 (SEQ ID No. 4) shown in Table 1.

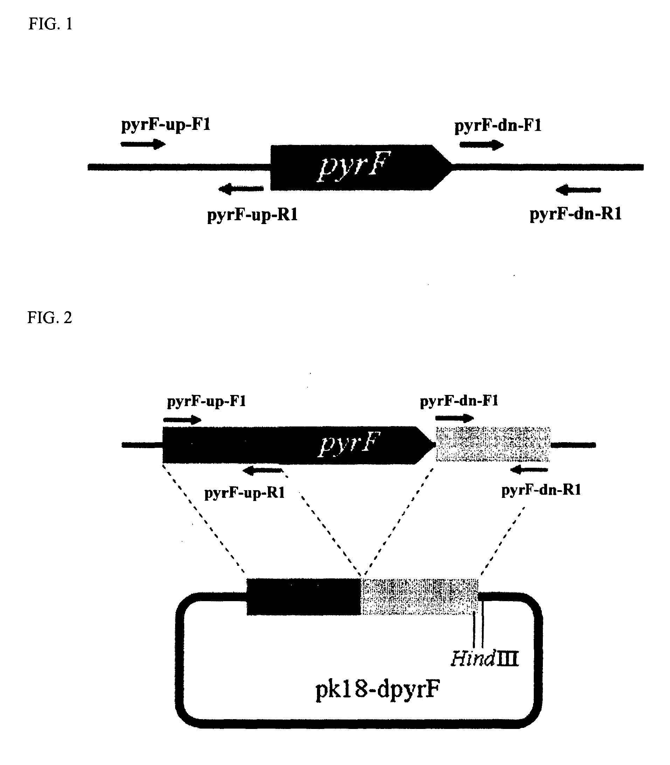

As shown in Figs. 1 and 2, the primers of the pyrF-uP-F1 (SEQ ID No. 1) and the pyrF-uP-R1 (SEQ ID No. 2) amplify a region adjacent to the upstream region of the pyrF gene, and the primers of the pyrF-dn-F1 (SEQ ID No. 3) and the pyrF-dn-R1 (SEQ ID No. 4) amplify a region adjacent to the downstream region of the pyrF gene.

**[Table 1]**

| Primer used for constructing pyrF gene-destroying vector | | |
|---|---|---|
| SEQ ID | Name of Primer | Sequence (5'to3') |
| | | Supplementary explanation |
| No. 1 | pyrF-uP-F1 | tgacgttctagaccctacctctccaagattacc |
| | | Restriction enzyme Xbal site added |
| No. 2 | pyrF-uP-R1 | tgacgtactagtggcaagcaggccagaag |
| | | Restriction enzyme SpeI site added |
| No. 3 | pyrF-dn-F1 | tagcgtactagtaacttcggcctgctttcatgc |
| | | Restriction enzyme SpeI site added |
| No. 4 | pyrF-dn-R1 | tgacctgatatctgtccaagcttatgcaccttcc |
| | | Restriction enzyme EcoRV added |

**[Table 2]**

| | | | |
|---|---|---|---|
| KOD-Plus-Neo (Product of TOYOBO Co., Ltd.) | 1µl | | |
| | | 94°C 2min. | 1cycle |
| 10 x buffer | 5µl | 98°C 10sec. | |
| 2mM dNTPs | 5µl | 55°C 30sec. | 25cycle |
| 25mM MgSO₄ | 3µl | 68°C 45sec. | |
| Primer F (10µM) | 1.5µl | 4°C ∞ | 1 cycle |
| Primer R (10µM) | 1.5µl | | |
| Template | 3µl | | |
| Sterilized water | 30µl | | |

After a PCR product obtained was treated with a restriction enzyme SpeI, the product was refined using an MagExtractor Kit (Product of TOYOBO Co., Ltd.), 5µl of a PCR product of a pyrF gene upstream region, 5µl of a PCR product of a downstream region and 10µl of Ligation high ver.2 (Product of TOYOBO Co., Ltd.) were mixed and incubated at 16°C for 30 minutes. By using a ligation product as a template, PCR was performed on the conditions shown in Table 3 by using the primers of the pyrF-uP-F1 (SEQ ID No. 1) and the pyrF-dn-R1 (SEQ ID No. 4).

**[Table 3]**

| | | | |
|---|---|---|---|
| KOD-Plus-Neo (Product of TOYOBO Co., Ltd.) | 1µl | 94°C 2min. | 1cycle |
| | | 98°C 10sec. | |
| 10 x buffer | 5µl | 55°C 30sec. | 20cycle |
| 2mM dNTPs | 5µl | 68°C 1min 15sec. | |
| 25mM MgSO₄ | 3µl | 4°C ∞ | 1cycle |
| Primer F (10µM) | 1.5µl | | |
| Primer R (10µM) | 1.5µl | | |
| Template | 2µl | | |
| Sterilized water | 31µl | | |

After a PCR product obtained was subjected to gel extraction by using an MagExtractor Kit (Product of TOYOBO Co., Ltd.), 2µl of a SmaI-treated plasmid pk18mob was mixed with 8µl of a gel-extracted PCR product and 10µl of a Ligation high ver.2 (Product of TOYOBO Co., Ltd.) and the product was incubated at 16°C for one hour. 10µl of a ligation solution was added to an Escherichia coli HST08 Premium competent cell (product of Takara Bio Inc.) to be slowly agitated, allowed to stand in ice water for 10 minutes, subjected to heat shock at 42°C for 1 minute and was immediately allowed to stand in ice water.

1ml of an SOC medium was added thereto and the product was incubated at 37°C for 1 hour. The product was smeared on an LB agar medium (kanamycin, X-Gal, IPTG-containing) and cultured at 37°C overnight to obtain a grown colony.

### [Confirmation of pyrF gene-destroying-vector]

After transplanting the grown colony above (construction of pyrF gene-destroying-vector pk18-dpryF) to a kanamycin-added LB medium, colony direct PCR was performed to confirm an insert. The primers used were a pyrF-uP-F1 (SEQ ID No. 1) and a pyrF-dn-R1 (SEQ ID No. 4) shown in Table 1. Table 4 shows the conditions of the colony direct PCR.

**[Table 4]**

| | | | |
|---|---|---|---|
| Sapphire Amp Master Mix (Product of Takara Bio Inc.) | 10µl | 94°C 1min. | 1 cycle |
| | | 98°C 5sec. | |
| Primer F (10µM) | 0.5µl | 55°C 5sec. | 30cycle |
| Primer R (10µM) | 0.5µl | 72°C 30sec. | |
| Template | colony | 4°C ∞ | 1cycle |
| Sterilized water | 9µl | | |

In a PCR product obtained, a band was confirmed by electrophoresis.

A strain whose band was confirmed was cultured with a kanamycin-added LB liquid medium overnight to extract a plasmid.

After concentration measurement by absorbance and band confirmation by electrophoresis, a base sequence was decoded by sequence to confirm the construction of a targeted pyrF gene-destroying-vector pk18-dpryF.

### 1.2. Preparation of pyrF gene-destroying strain (ΔpyrF strain) of M. thermoacetica ATCC 39073 strain

The pyrF gene-destroying-vector pk18-dpryF constructed in the above 1.1 was introduced to the M. thermoacetica ATCC 39073 strain according to the following procedures, and a strain in which a pyrF gene is destroyed (ΔpyrF strain) by homologous recombination of double cross-over was selected.

### [Introduction of pyrF gene-destroying-vector pk18-dpryF to M. thermoacetica ATCC 39073 strain]

An HS buffer, composed of 272mM sucrose and 16mM HEPES, was prepared using potassium hydroxide so that pH was 7, boiled for 20 minutes and substituted with N₂ gas for 20 minutes.

An M. thermoacetica ATCC39073 strain was cultured with a modified ATCC 1754 PETC medium with a mixed gas (80% hydrogen and 20% carbon dioxide) as a substrate or a modified ATCC 1754 PETC medium to which glycine was added with a final concentration of 5g/L.

The M. thermoacetica ATCC39073 strain was cultured until the bacterial cell concentration was approx. 0.3 at OD₆₀₀, approx. 100ml of a culture solution was harvested and a bacterial cell was washed with an HS buffer twice.

The bacterial cell washed was suspended in an approximate amount of a HS buffer (approx. 3ml) and mixed with 380µl of a suspension and 20µl of a plasmid.

Electroporation was performed on a condition of 1.5kv, 500Ω, 50µF or 2.0kv, 500Ω, 50µF by using a Bio-Rad Gene Pulser (registered trademark) and a cuvette with a gap of 0.2cm (Product of Bio-Rad Laboratories, Inc.).

A suspension obtained after electroporation was inoculated in 5ml of a medium to which pyruvic acid was added with a final concentration of 40mM. 2 days after culturing at 55°C, the product was inoculated in an agar medium to which uracil and 5-fluoroorotic acid (5-FOA) were added with final concentrations of 10µg/ml and 0.2%, respectively and a roll tube was prepared.

### [Confirmation of pyrF gene-destroying strain (ΔpyrF strain) by direct PCR]

20 colonies formed in the above agar medium were inoculated in a liquid medium to which 5ml uracil and 5-FOA were added with final concentrations of 10µg/ml and 0.2%, respectively, and, 6 strains whose medium was suspended on the 3^{rd} day after culturing were selected to harvest 1ml of a culture solution.

The product was suspended with 20µl of a TE buffer containing Acromopeptidase (20mg/ml) + lysozyme (20mg/ml), incubated at 37°C for 5 minutes, and 20µl of DMSO was added thereto and suspended to be defined as a PCR template.

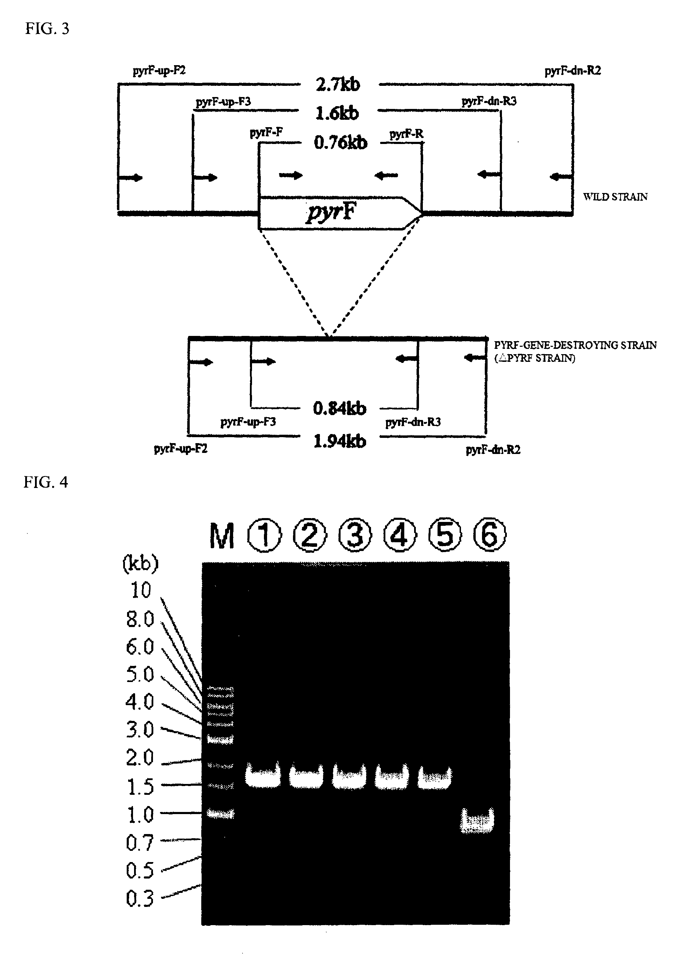

Colony direct PCR was performed on the conditions shown in Table 6 by using the primer combinations shown in Table 5: a pyrF-uP-F2 (SEQ ID No. 5) and a pyrF-dn-R2 (SEQ ID No. 6), a pyrF-uP-F3 (SEQ ID No. 7) and a pyrF-dn-R3 (SEQ ID No. 8), and a pyrF-F (SEQ ID No. 9) and a pyrF-R (SEQ ID No. 10). A band was confirmed by electrophoresis. Fig. 3 shows the location of each primer and the length of an expected DNA fragment. Fig. 3 shows the location of each primer and the length of an expected DNA fragment.

**[Table 5]**

| Primer used for confirming pyrF-gene-destroying strain (ΔpyrF strain) | | |
|---|---|---|
| SEQ ID No. | Name of Primer | sequence (5' to 3') |
| | | Supplementary explanation |
| 5 | pyrF-up-F2 | accctacctctccaagattacc |
| 6 | pyrF-dn-R2 | tgtccaagcttatgcaccttcc |
| 7 | pyrF-uP-F3 | tgtcctcaacaccctcacc |
| 8 | pyrF-dn-R3 | tcttcccaggtcctgtagg |
| 9 | pyrF-F | acctgaagttccacgacatcc |
| 10 | pyrF-R | ggtcacgatgacgaactc |

**[Table 6]**

| | | | |
|---|---|---|---|
| KOD-FX (Product of TOYOBO Co., Ltd.) | 1µl | 94°C 2min. | 1 cycle |
| 2 x buffer | 25µl | 98°C 10sec. | |
| dNTPs | 10µl | 54°C 30sec. | 30cycle |
| Primer F (10µM) | 1.5µl | 68°C 3min.30sec. | |
| Primer R (10µM) | 1.5µl | 4°C ∞ | 1cycle |
| Template | 2µl | | |
| Sterilized water | 10µl | | |

### <Evaluation>

Several attempts to form a colony by roll tube method obtained many colonies. 20 strains were selected therefrom to be cultured in a liquid medium (10µg/ml uracil and 0.2% 5-FOA). A bacterial cell was harvested from a culture solution in which growth of the bacterial cell was confirmed and confirmed by direct PCR.

Primer combinations: a pyrF-uP-F2 and a pyrF-dn-R2, and a pyrF-uP-F3 and a pyrF-dn-R3 were used in 6 strains that showed proliferation on the 3^{rd} day after culturing. PCR from outside a pyrF found that a band was confirmed shorter than a wild strain in one out of 6 strains.

In addition, a primer combination: a pyrF-F and a pyrF-R was used to perform PC-R inside a pyrF. It found no band in the above strains.

From these observations, it was estimated that strains whose band is shorter than wild strains are likely to correspond to a pyrF gene-destroying strain (ΔpyrF strain), and another PCR was performed after chromosome extraction. As a result, a band pattern which was found like in direct PCR was obtained.

Furthermore, since a pyrF gene-destroyed portion is imparted with one restriction enzyme SpeI site (primer pyrF-up-R1 and pyrF-dn-F1), an SpeI site was cleaved to confirm 2 bands when the above PCR product was treated with SpeI.

Subsequently, a pyrF gene-destroying potential strain was subjected to uracil requiring property test. A pyrF gene-destroying strain was inoculated in a modified ATCC 1754 PETC medium excluding yeast extract, and proliferation was confirmed when uracil was added with a final concentration of 10µg/ml and was not added.

As a result, bacterial cell proliferation was confirmed in a uracil-added sample on the 2^{nd} day after culturing, while proliferation was not confirmed in a no-uracil-added sample. Consequently, it was confirmed that a pyrF gene is destroyed.

The pyrF gene-destroying strain is under national deposit as an MTA-D-pF strain (accession number: NITEP-1057) at NITE Patent Microorganisms Depositary (NPMD). The national deposit will be transferred to international deposit on June 1, 2012, and NPMD issues the certification "Notice of acceptance of biological genetic resources" as of this date. The international deposit accession number will be NITE BP-1057.

### 2. Complementarity test 1 of M. thermoacetica ATCC 39073 pyrF gene-destroying strain (ΔpyrF strain)

### 2.1. Construction of pyrF gene complementary vector (gene-expression vector)

A pyrF gene complementary vector was constructed in order to perform a complementarity test of an M. thermoacetica ATCC 39073 pyrF gene-destroying strain (ΔpyrF strain) according to the following procedures.

### [Construction of pyrF gene complementary vector]

By using a primer combination: a primer pyrF-uP-F1 (SEQ ID No. 1) and a pyrF-dn-R1 (SEQ ID No. 4) shown in Table 1, PCR was perform on conditions shown in Table 7 to amplify a pyrF gene translational region, and approx. 2.7kbp of a gene fragment containing approx. 1000bp on 5' side and approx. 1000bp on 3' side.

**[Table 7]**

| | | | |
|---|---|---|---|
| KOD-FX (Product of TOYOBO Co., Ltd.) | 1µl | | |
| 2 x buffer | 25µl | | |
| dNTPs | 10µl | 94°C 2min. | 1cycle |
| Primer F (10µM) | 1.5µl | 98°C 10sec. | |
| Primer R (10µM) | 1.5µl | 54°C 30sec. | 30cycle |
| Template | 2µl | 68°C 3min 30sec. | |
| Sterilized water | 10µl | 4°C ∞ | 1cycle |

A PCR product obtained was subjected to gel extraction by using MagExtractor Kit (Product of TOYOBO Co., Ltd.).

2µl of a plasmid pBluescript II KS+ treated with EcoRV or 2µl of a plasmid pk18mob treated with SmaI were mixed with 8µl of a gel-extracted PCR product and 10µl of a Ligation high ver.2 (Product of TOYOBO Co., Ltd.) to be incubated at 16°C for 1 hour.

10µl of a ligation solution was added to an Escherichia coli HST08 Premium competent cell (Product of Takara Bio Inc.) to be lightly agitated, and was allowed to stand in ice water for 10 minutes. Thereafter, the product was subjected to heat shock at 42°C for 1 minute and immediately allowed to stand in ice water.

1ml of an SOC medium was added thereto and incubated at 37°C for 1 hour. When a pBluescript II KS+ is used, the product was smeared in an LB agar medium (ampicillin, X-Gal, IPTG-containing), and when a pk18mob was used, the product was smeared in an LB agar medium (kanamycin, X-Gal, IPTG-containing), and cultured at 37°C overnight to obtain a grown colony.

### [Confirmation of pyrF gene complementary vector]

After the above grown colony was transplanted to an ampicillin- or a kanamycin-added LB agar medium, colony direct PCR was performed to confirm an insert. The primers used were a pyrF-uP-F1 (SEQ ID No. 1) and a pyrF-dn-R1 (SEQ ID No. 4) shown in Table 1. Table 8 shows the conditions of colony direct PCR.

**[Table 8]**

| | | | |
|---|---|---|---|
| KOD-FX (Product of TOYOBO Co., Ltd.) | 1µl | 94°C 2min. | 1cycle |
| 2 x buffer | 25µl | 98°C 10sec. | |
| dNTPs | 10µl | 54°C 30sec. | 30cycle |
| Primer F (10µM) | 1.5µl | 68°C 3min 30sec. | |
| Primer R (10µM) | 1.5µl | 4°C ∞ | 1cycle |
| Template | 2µl | | |
| Sterilized water | 10µl | | |

Moreover, a strain in which a band was confirmed by electrophoresis was cultured with an ampicillin-added LB liquid overnight when a pBluescript II KS+ was used, and the strain was cultured with a kanamycin (pk18-epyrF)-added LB liquid overnight when a pk18mob was used to extract a plasmid.

After concentration measurement by absorbance and electrophoresis of an EcoRl- or a Pstl-treated sample, a base sequence was decoded by sequence to confirm the construction of targeted pyrF gene complementary vectors pk18-epyrF and pBS-epyrF.

### 2.2. Introduction of pyrF gene complementary vector pBS-epyrF to M. thermoacetica ATCC 39073 pyrF gene-destroying strain (ΔpyrF strain)

The pyrF gene complementary vector pBS-epyrF constructed in 2.1. was introduced to the M. thermoacetica ATCC 39073 pyrF gene-destroying strain (ΔpyrF strain) constructed in 1.2. to perform a complementarity test by homologous recombination according to the following procedures.

### [Introduction of pyrF gene complementary vector pBS-epyrF by electroporation]

An HS buffer, composed of 272mM sucrose and 16mM HEPES, was prepared using potassium hydroxide so that pH was 6.7, boiled for 20 minutes and substituted with N₂ gas for 20 minutes.

A ΔpyrF strain was cultured with a complete synthetic medium to which uracil was added with a final concentration of 10µg/ml with a mixed gas (80% hydrogen and 20% carbon dioxide) as a substrate.

The strain was cultured until the bacterial cell concentration was approx. 0.1 at OD₆₀₀, approx. 100ml of a culture solution was harvested and the product was washed with a 272mM sucrose buffer twice so that pH was 7 using potassium hydroxide.

The bacterial cell washed was suspended in an appropriate amount of an HS buffer (approx. 3ml) and mixed with 380µl of a suspension and 20µl of a plasmid.

Electroporation was performed on a condition of 1.5kv, 500Ω, 50µF or 2.0kv, 500Ω, 50µF by using a Bio-Rad Gene Pulser (registered trademark) and a cuvette with a gap of 0.2cm (Product of Bio-Rad Laboratories, Inc.).

A suspension obtained after electroporation was inoculated in 5ml of a complete synthetic medium to which uracil and pyruvic acid were added with final concentrations of 10µg/ml and 40mM, respectively, cultured at 55°C for 2 days, washed in a complete synthetic medium, inoculated in a medium containing a complete synthetic medium and an agar and a roll tube was prepared.

### [Confirmation of pyrF gene complementary strain by direct PCR]

The colony obtained in the above procedures was inoculated in 5ml of a complete synthetic medium, samples having a suspended medium on the 4^{th} day after culturing were selected to harvest 1ml of a culture solution.

The product was suspended with 10µl of a TE buffer containing Acromopeptidase (20mg/ml) + lysozyme (20mg/ml), incubated at 37°C for 5 minutes, and 10µl of DMSO was added thereto and suspended to be defined as a PCR template.

Colony direct PCR was performed on the conditions shown in Table 9 by using the primer combination shown in Table 5: a pyrF-uP-F3 (SEQ ID No. 7) and a pyrF-dn-R3 (SEQ ID No. 8).

A band was confirmed by electrophoresis to determine a pyrF gene complementary strain was obtained.

**[Table 9]**

| | | | |
|---|---|---|---|
| KOD-FX (Product of TOYOBO Co., Ltd.) | 1µl | | |
| 2 x buffer | 25µl | 94°C 2min. | 1cycle |
| dNTPs | 10µl | 98°C 10sec. | |
| Primer F (10µM) | 1.5µl | 54°C 30sec. | 30cycle |
| Primer R (10µM) | 1.5µl | 68°C 2min 10sec. | |
| Template | 2µl | 4°C ∞ | 1cycle |
| Sterilized water | 10µl | | |

### <Evaluation>

A band in 4 sample strains whose culture solution was white-turbid on the 4^{th} day after culturing was confirmed by direct PCR. Fig. 4 shows the results.

The electrophoresis shown in Fig. 4 found that lanes 1 to 4 correspond to a complementary strain, lane 5 corresponds to a wild strain and lane 6 corresponds to a pyrF-destroying strain. A band was shown in all the 4 strains at the same position of approx. 1.6kbp as a wild strain. This observation means that a complementary plasmid is incorporated into a cell of a pyrF gene-destroying strain by electroporation and a pyrF is inserted into an original position by homologous recombination. In the pyrF-destroying strain, the length of a band was an expected value at approx. 0.9 bp.

### 3. Complementarity test 2 of M. thermoacetica ATCC 39073 pyrF gene-destroying strain (ΔpyrF strain)

### 3.1. Construction of complementary vector for inserting transforming-DNA

When a ΔpyrF strain was subjected to a pyrF gene complementarity test, part of a transforming-gene was inserted into a pyrF upstream region to construct a plasmid whose chromosome can be processed by homologous recombination. Specifically, part of a lacZ gene of a Thermoanaerobacter ethanolicus 39E strain (approx. 500bp) was inserted into a pyrF gene upstream region of the pyrF gene complementary vector pk18-epyrF constructed in 2.1. The procedures are shown as follows.

### [Construction of complementary vector for inserting transforming-DNA by In-Fusion PCR]

A vector region containing a pyrF gene region was PCR-amplified by using primer combinations: a primer pyrF-1-R (SEQ ID No. 11) and a pyrF-1-F (SEQ ID No. 12), a pyrF-2-R (SEQ ID No. 13) and a pyrF-2-F (SEQ ID No. 14), and a pyrF-3-R (SEQ ID No. 15) and a pyrF-3-F (SEQ ID No. 16) shown in Table 10. Table 11 shows PCR conditions for each combination.

**[Table 10]**

| Primer used | | | |
|---|---|---|---|
| SEQ ID No. | Primer F | Sequence | Target Gene |
| | Primer R | Sequence | |
| 11 | pyrF-1-R | agactaacaacttcaaggggtacatactcctgcag | |
| 12 | pyrF-1-F | cagtggttactgacatcccgtccttaacggagg | |
| 13 | pyrF-2-R | agactaacaacttcagctcctccgttaaggacg | |
| 14 | pyrF-2-F | cagtggttactgacacttcaagcaccaggcgag | |
| 15 | pyrF-3-R | agactaacaacttcactcgcctggtgcttgaag | |
| 16 | pyrF-3-F | cagtggttactgacaggaagggaagggcttttcc | |
| 17 | lacZ-500-F | tgaagttgttagtcttcctcatgc | laeZ (approx.500bp) |
| 18 | lacZ-500-R | gtcagtaaccactgatgaacac | |

**[Table 11]**

| | | | |
|---|---|---|---|
| KOD-FX (Product of TOYOBO Co., Ltd.) | 1µl | 94°C 2min. | 1cycle |
| 2 × buffer | 25µl | 98°C 10sec. | 20cycle |
| dNTPs | 10µl | 54°C 30sec. | |
| Primer F (10µM) | 1.5µl | 68°C 6min 40sec. | |
| Primer R (10µM) | 1.5µl | 4°C ∞ | 1 cycle |
| Template | 1µl | | |
| Sterilized water | 10µl | | |

A lacZ gene region was PCR-amplified by using a primer LacZ-500-F (SEQ ID No. 17) and a LacZ-500-R (SEQ ID No. 18) shown in Table 10. Table 12 shows the PCR conditions.

**[Table 12]**

| | | | |
|---|---|---|---|
| Prime Star MAX (Product of Takara Bio Inc.) | 50µl | 94°C 10sec. | 1cycle |
| Primer F (10µM) | 3µl | 98°C 10sec. | |
| Primer R (10µM) | 3µl | 54°C 10sec. | 25cycle |
| Template | 1µl | 72°C 20sec. | |
| Sterilized water | 41µl | 4°C ∞ | 1cycle |

Each PCR product was subjected to gel extraction, and In-Fusion PCR (In-Fusion·Advantage PCR Cloning Kit, Product of Takara Bio Inc.) was performed on the conditions shown in Table 13.

**[Table 13]**

| | |
|---|---|
| Plasmid (PCR product) | 2µl |
| Insert | 4µl |
| Sterilized water | 1µl |
| In-Fusion buffer | 2µl |
| In-Fusion Enzyme | 11µl |
| 37°C for 30 minutes → 50°C for 15 minutes | |

50µl of sterilized water was added to an In-Fusion sample to be diluted, and 10µl of a diluted sample was used for transformation.

Colonies were selected by direct PCR.

### <Evaluation>

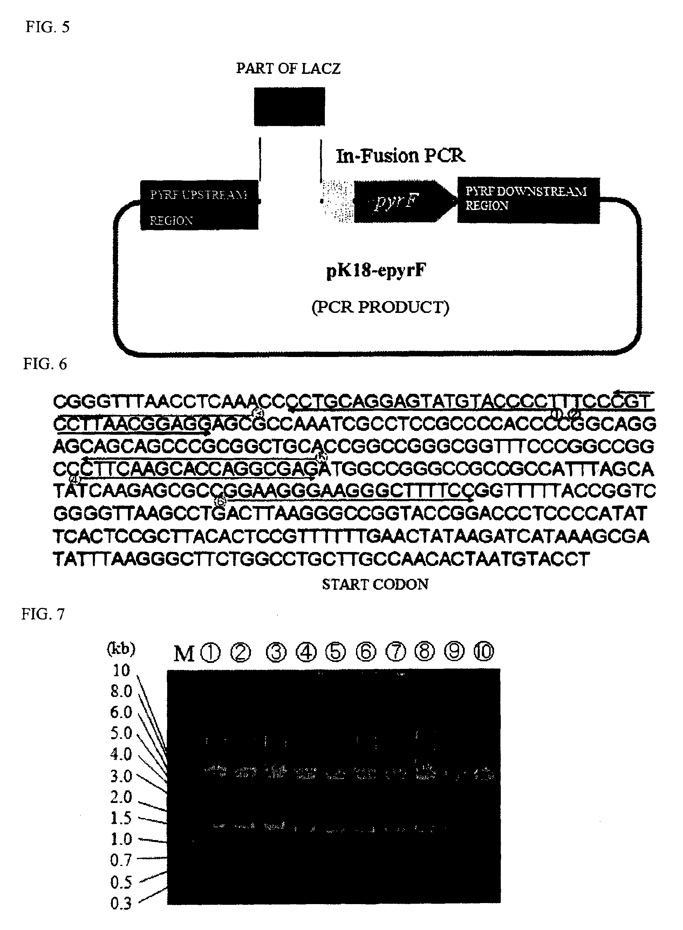

In order to insert part of a lacZ gene to a pyrF upstream region, inverse PCR was performed by using primer combinations: a primer pyrF-1-R and a pyrF-1-F, pyrF-2-R and a pyrF-2-F, and a pyrF-3-R and a pyrF-3-F with the pk18-epyrF constructed in 2.1. as a template. Accordingly, the position of a primer is shifted so that a promoter region comes at 300bp, 203bp, 147bp to obtain 3 patterns of PCR products (Figs. 5 and 6). Moreover, in order to insert a lacZ gene to a plasmid by using In-Fusion PCR, a lacZ gene and a homologous sequence were added to each primer. The underlined sequences of SEQ ID No. 11 to 16 in Table 10 correspond to promoter regions. Non-underlined sequences determine the position of a primer, and SEQ ID No. 11 corresponds to a circled number 1 in Fig. 6, SEQ ID No. 12 corresponds to a circled number 2 in Fig. 6, SEQ ID No. 13 corresponds to a circled number 3 in Fig. 6, SEQ ID No. 14 corresponds to a circled number 4 in Fig. 6, SEQ ID No. 15 corresponds to a circled number 5 in Fig. 6 and SEQ ID No. 16 corresponds to a circled number 6 in Fig. 6 (each having arrow).

In-Fusion PCR and transformation in E. coli obtained a plurality of colonies. Colony direct PCR with a LacZ-500-F and a LacZ -500-R as a primer found a targeted band in all the strains.

3 strains out thereof were cultured as a group to extract a plasmid. A plasmid composed of primers of a pyrF-1-R and a pyrF-1-F is defined as pk18-pyz-1, a plasmid composed of primers of a pyrF-2-R and a pyrF-2-F is defined as pk18-pyz-2, and a plasmid composed of primers of a pyrF-3-R and a pyrF-3-F is defined as pk18-pyz-3.

After confirming a band treated with a restriction enzyme KpnI, an insert of lacZ gene was confirmed in all the strains as shown in Fig. 7.

In electrophoresis observations in Fig. 7, lanes 1, 2 and 3 correspond to a promoter region 300bp (pk18-pyz-1), lanes 4, 5 and 6 correspond to a promoter region 203bp (pk18-pyz-2), and lanes 7, 8 and 9 correspond to a promoter region 147bp (pkI8-pyz-3). Lane 10 corresponds to a pk18-epyrF.

### 3.2. Introduction of complementary vector for inserting transforming-DNAs pk18-pyz-1, pk18-pyz-2 and pk18-pyz-3 to M. thermoacetica ATCC 39073 pyrF gene-destroying strain (ΔpyrF strain)

The complementary vectors for inserting a transforming-DNA (pk18-pyz-1, pk18-pyz-2 and pk18-pyz-3) constructed in 3.1. were introduced to the M. thermoacetica ATCC 39073 pyrF gene-destroying strain (ΔpyrF strain) constructed in 1.2. according to the following procedures to perform a complementarity test by homologous recombination.

### [Introduction of complementary vectors for inserting a transforming-DNA (pk18-pyz-1, pk18-pyz-2 and pk18-pyz-3) by electroporation]

An HS buffer, composed of 272mM sucrose and 16mM HEPES, was prepared by using potassium hydroxide so that pH was 6.7, boiled for 20 minutes and substituted with N₂ gas for 20 minutes.

A ΔpyrF strain was cultured in a complete synthetic medium to which uracil was added with a final concentration of 10µg/ml with a mixed gas (80% hydrogen and 20% carbon dioxide) as a substrate.

The strain was cultured until the bacterial cell concentration was approx. 0.1 at OD₆₀₀, approx. 50ml of a culture solution was harvested and the product was washed with a 272mM sucrose buffer twice so that pH was 7 using potassium hydroxide, and the bacterial cell washed was suspended in an appropriate amount of an HS buffer (approx. 3ml).

380µl of a suspension and 5 to 10µl of a plasmid were mixed and electroporation was performed on a condition of 1.5kv, 500Ω, 50µF, or 2.0kv, 500Ω, 50µF by using a Bio-Rad Gene Pulser (registered trademark) and a cuvette with a gap of 0.2cm (Product of Bio-Rad Laboratories, Inc.).

A suspension after electroporation was inoculated in 5ml of a complete synthetic medium to which uracil was added with a final concentration of 10µg/ml, cultured with a mixed gas (80% hydrogen and 20% carbon dioxide) as a substrate, cultured at 55°C for 2 days, washed in a complete synthetic medium, inoculated in a medium containing a complete synthetic medium and an agar and a roll tube was prepared.

### [Confirmation of pyrF gene complementary strain by direct PCR]

The colony obtained in the above procedures was inoculated in 5ml of a complete synthetic medium, samples having a suspended medium after culturing were selected to harvest 1ml of a culture solution.

The product was suspended with 10µl of a TE buffer containing Acromopeptidase (20mg/ml) + lysozyme (20mg/ml), incubated at 37°C for 5 minutes, and 10µl of a DMSO was added thereto and suspended to be defined as a PCR template.

Colony direct PCR was performed on the conditions shown in Table 14 by using the primer combination shown in Table 5: a pyrF-uP-F3 (SEQ ID No. 7) and a pyrF-dn-R3 (SEQ ID No. 8).

A band was confirmed by electrophoresis to determine a pyrF gene complementary strain was obtained.

**[Table 14]**

| | | | |
|---|---|---|---|
| KOD-FX (Product of TOYOBO Co., Ltd.) | 1µl | 94°C 2min. | 1cycle |
| 2 x buffer | 25µl | 98°C 10sec. | |
| dNTPs | 10µl | 54°C 30sec. | 30cycle |
| Primer F (10µM) | 1.5µl | 68°C 3min 30sec. | |
| Primer R (10µM) | 1.5µl | 4°C ∞ | 1cycle |
| Template | 2µl | | |
| Sterilized water | 10µl | | |

### <Evaluation>

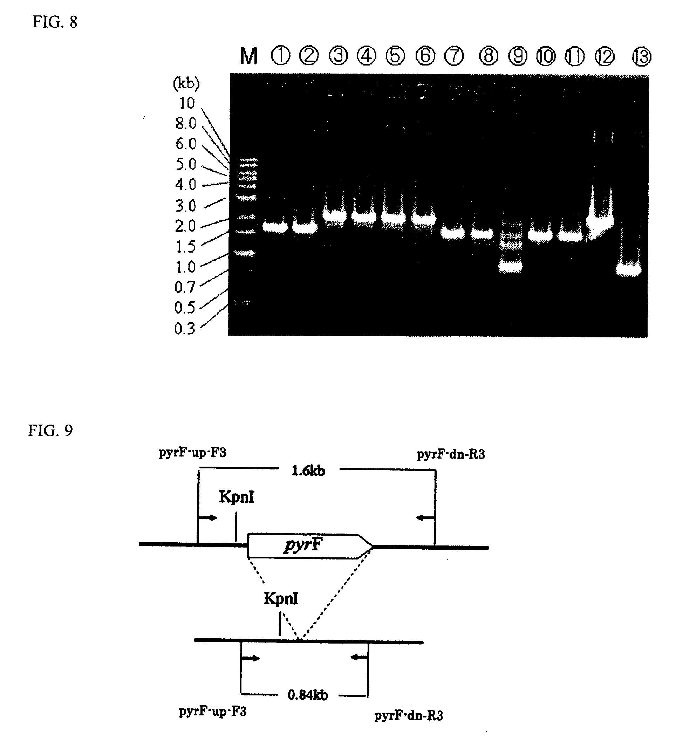

20 colonies were obtained in total, and 8 strains out thereof were subjected to band confirmation by direct PCR (Fig. 8).

The electrophoresis shown in Fig. 8 found that lanes 1 to 8 correspond to a colony isolated strain, lane 9 corresponds to a ΔpyrF strain direct, lane 10 corresponds to an ATCC39073 wild strain direct, lane 11 corresponds to an extract DNA of an ATCC39073 wild strain, lane 12 corresponds to a complementary vector for inserting a transforming-DNA pk18-pyz-1 and lane 13 corresponds to a pyrF gene-destroying-vector pk18-dpryF.

As shown in Fig. 8, a band was shown in 4 strains (lanes 3, 4, 5 and 6) at the position of approx. 2.1kbp, bigger than the wild strains (lanes 10 and 11), and introduction of a LacZ gene of approx. 500bp (transforming-gene) was confirmed. In the remaining 4 strains, a band was shown at the same position of approx. 1.6kbp as the wild strains.

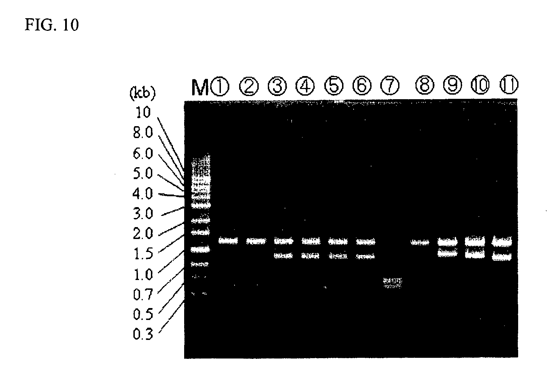

Subsequently, PCR products of 6 strains were refined, and treated with a restriction enzyme KpnI confirm a band by electrophoresis. The site of a restriction enzyme KpnI is located at the position in Fig. 9. A PCR product treated with KpnI showed two bands, but a band in a downstream region appeared at the larger position than the wild strains when a transforming-gene was inserted. Fig. 10 shows the results.

In Fig. 10, lanes 1 to 6 correspond to a colony isolated strain, lane 7 corresponds to a ΔpyrF strain direct, lane 8 corresponds to an extract DNA of an ATCC39073 wild strain, lane 9 corresponds to a complementary vector for inserting a transforming-DNA pk18-pyz-1, lane 10 corresponds to a complementary vector for inserting a transforming-DNA pk18-pyz-2 and lane 11 corresponds to a complementary vector for inserting a transforming-DNA pk18-pyz-3.

As shown in Fig. 10, lanes 1 and 2 showed the same band as the wild strains, lanes 3, 4, 5 and 6 showed a band at the larger position than the wild strains, and an insert of a transforming-gene LacZ was confirmed.

In addition, Total DNA was extracted from a bacterial cell obtained not by direct PCR but by proliferation. After PCR on similar conditions confirmed an insert of a transforming-gene LacZ, the results were the same as direct PCR.

## Claims

1. A primer set used for creating a uracil requiring strain obtained by deleting or destroying a gene coding for orotidine-5-phosphate decarboxylase in Moorella bacteria by homologous recombination, wherein
the primer set is represented by SEQ ID No. 1 and 2 that amplify an upstream region adjacent to said gene coding for orotidine-5-phosphate decarboxylase.

2. A primer set used for creating a uracil requiring strain obtained by deleting or destroying a gene coding for orotidine-5-phosphate decarboxylase in Moorella bacteria by homologous recombination, wherein,
the primer set is represented by SEQ ID No. 3 and 4 that amplify a downstream region adjacent to said gene coding for orotidine-5-phosphate decarboxylase.

3. A primer set used for creating a uracil requiring strain obtained by deleting or destroying a gene coding for orotidine-5-phosphate decarboxylase in Moorella bacteria by homologous recombination, wherein
the primer set comprises: a primer that is represented by SEQ ID No. 1 and 2 that amplify an upstream region adjacent to said gene coding for orotidine-5-phosphate decarboxylase; and a primer that is represented by SEQ ID No. 3 and 4 that amplify a downstream region adjacent to said gene coding for orotidine-5-phosphate decarboxylase.

4. A method for homologous recombination, comprising creating a uracil requiring strain obtained by deleting or destroying a gene coding for orotidine-5-phosphate decarboxylase in Moorella bacteria by homologous recombination by using the primer set according to any of claims 1 to 3.
